Europäisches Patentamt

European Patent Office

Office européen des brevets

(11) Veröffentlichungsnummer: **0 183 214**
A2

(19)

(12) **EUROPÄISCHE PATENTANMELDUNG**

(21) Anmeldenummer: 85114916.1

(22) Anmeldetag: 25.11.85

(51) Int. Cl.⁴: **C 07 G 11/00**
C 12 P 1/06, A 61 K 35/70
A 23 K 1/17

(30) Priorität: 30.11.84 DE 3443681

(43) Veröffentlichungstag der Anmeldung:
04.06.86 Patentblatt 86/23

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Bischoff, Erwin, Dr.
Pahlkestrasse 73
D-5600 Wuppertal 1(DE)

(72) Erfinder: Müller, Hartwig, Dr.
Steinstrasse 15
D-5620 Velbert(DE)

(72) Erfinder: Salcher, Olga, Dr.
Am Eckbusch 43/100
D-5600 Wuppertal 1(DE)

(72) Erfinder: Berschauer, Friedrich, Dr.
Claudiusweg 9
D-5600 Wuppertal 1(DE)

(72) Erfinder: Scheer, Martin, Dr.
Herberts-Katernberg 7
D-5600 Wuppertal 1(DE)

(72) Erfinder: de Jong, Anno, Dr.
Stockmannsmühle 46
D-5600 Wuppertal 1(DE)

(72) Erfinder: Frobel, Klaus, Dr.
Birkenhöhe 5
D-5600 Wuppertal 1(DE)

(54) Organisch-chemische Verbindung, mikrobiologische Verfahren zu ihrer Herstellung sowie ihre Verwendung.

(57) Die vorliegende Erfindung betrifft eine neue organisch chemische Verbindung herstellbar in einem mikrobiologischen Verfahren mit Hilfe des Streptomyces Stammes BA 9 (gemäß DSM Nr. 3025).

Die erfindungsgemäße Verbindung ist wirksam gegen gram-positive und gram-negative Keime, sie eignet sich außerdem als Futterzusatzmittel.

EP 0 183 214 A2

Croydon Printing Company Ltd.

BAYER AKTIENGESELLSCHAFT        5090 Leverkusen, Bayerwerk
Konzernverwaltung RP
Patentabteilung              Rt/Kü-c  29. NOV. 1984

Organisch-chemische Verbindung, mikrobiologische
Verfahren zu ihrer Herstellung sowie ihre Verwendung

Die vorliegende Erfindung betrifft eine neue organisch-chemische Verbindung, mikrobiologische Verfahren zu ihrer Herstellung sowie ihre Verwendung
als Antibiotikum und als Mittel zur Förderung des
Wachstums und zur Steigerung der Futterverwertung
bei Tieren.

Die erfindungsgemäße Verbindung läßt sich durch die folgenden Eigenschaften und Parameter charakterisieren:

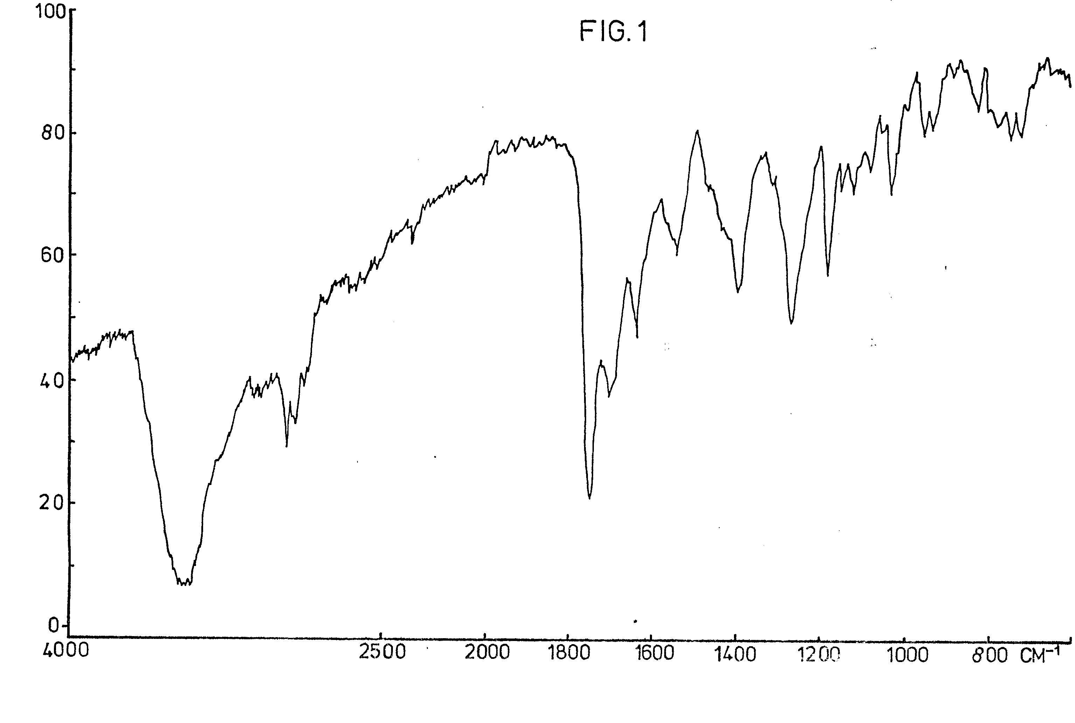

1.  IR-KBr-Absorptionsspektrum des natriumhaltigen
    Antibiotikums gemäß Abb. 1 (Abszisse: Wellen-
    zahl in $cm^{-1}$, Ordinate Absorption).

    Es zeigt, bei folgenden Wellenlängen (ausge-
    drückt in $cm^{-1}$) charakteristische Absorptions-
    banden:

|      |      |
|------|------|
| 3456 | 1265 |
| 2960 | 1178 |
| 2910 | 1145 |
| 1750 | 1115 |
| 1700 | 1030 |

Le A-23 433

|      |     |
|------|-----|
| 1635 | 950 |
| 1540 | 930 |
| 1395 | 825 |

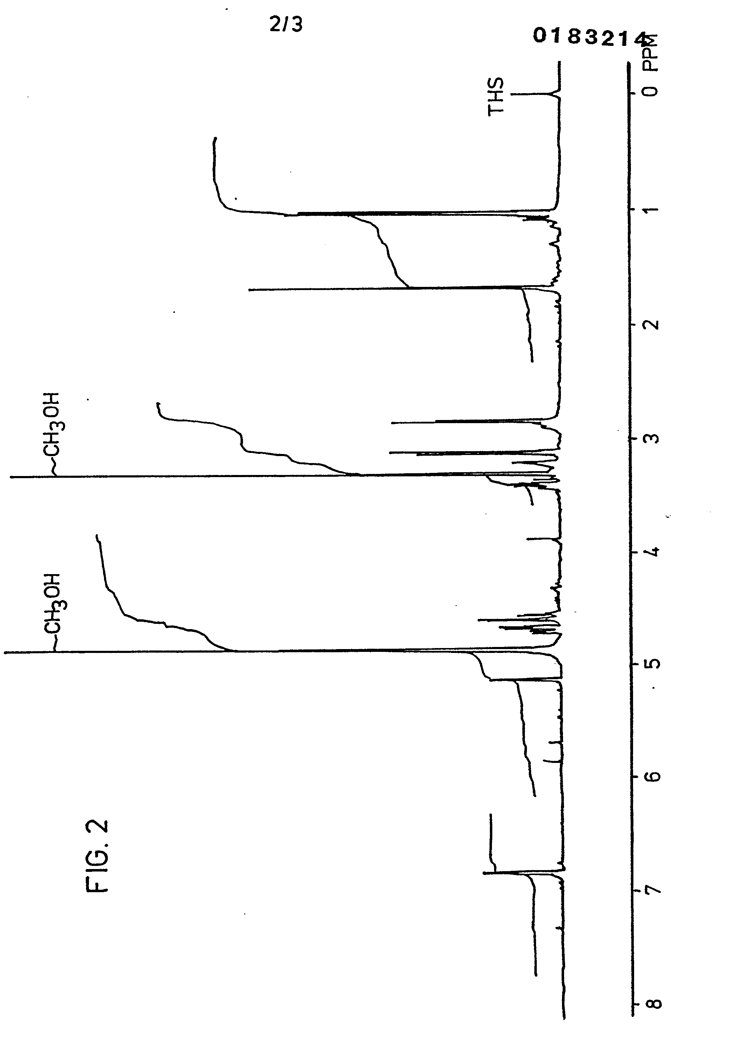

2. $^1$H-Kernresonanzspektrum, gemäß Abb. 2, angegeben in Teilen pro Million und Schwingungen pro Sekunde.

Es wurde mit einer Feldstärke von 300 MHz an einer Lösung des Antibiotikums in deuterisiertem Methanol mit Tetramethylsilan in deuterisiertem Methanol als innerem Standard aufgenommen.

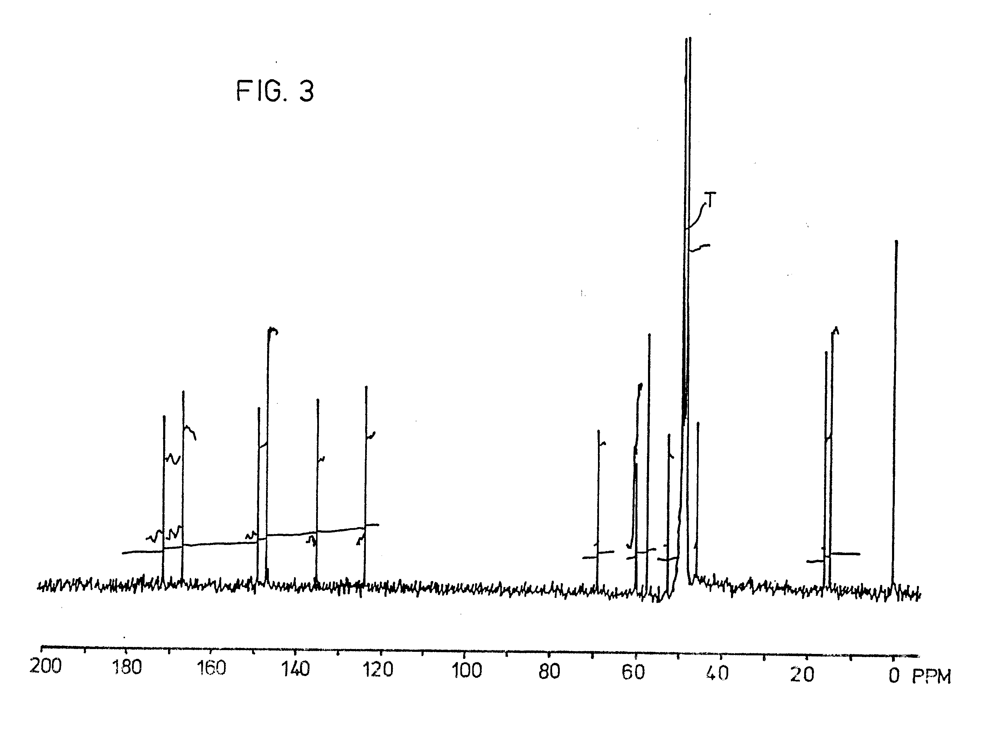

3. Das 13C-Kernresonanzspektrum gemäß Abb. 3. Es wurde in derselben Lösung wie das $^1$H-Spektrum an bei einer Feldstärke von 75,48 MHz gemessen.

4. Elementaranalyse (nach 2 Tagen Trocknen im Hochvakuum bei 30°C): C 61,1-62,3 %; H 5,2-6,4 %; O 27,5-30,0 %.

5. Summenformel: $(C_{14-16} H_{16-20} O_{4-6})_n$.

Es muß hier darauf hingewiesen werden, daß bei höhermolekularen Naturstoffen die Fehlerbreite der Elementaranalyse größer sein kann als allgemein üblich; daher ist eine genaue Bestimmung der Summenformel nicht möglich (R.B. Woodward, Angew. Chem. 69, 50-51 (1957)).

Le A 23 433

6.  Summenformel der Na-haltigen Verbindung:
    $(C_{29-31}, H_{33-37}, O_{9-11}, Na)_n$.

7.  Die Verbindung ist gut löslich in Essigsäureethylester, Chloroform, MeOH und niedrigen
    Alkoholen; schlecht löslich in Wasser.

8.  Die Verbindung läßt sich nach Chromatographie
    auf Kieselgel-Dünnschichtplatten mit
    $FeCl_3/H_2SO_4$ anfärben. Das Reagenz wurde nach
    den üblichen Vorschriften (vgl. E. Stahl,
    Dünnschichtchromatographie, 2. Aufl., Springer
    Verlag, Berlin, Heidelberg, New York 1967)
    hergestellt.

9.  Die antibakterielle Wirkung der Verbindung ist in
    Tabelle 1 wiedergegeben.

Des weiteren betrifft die vorliegende Erfindung neue
Mikroorganismen der Familie Streptomycetaceae, die bei
der Kultivierung in einem Kohlenstoff- und Stickstoffquellen sowie Mineralsalze enthaltenden Nährmedium
eine Verbindung produzieren, welche die oben unter
(1) bis (9) aufgeführten Eigenschaften und Parameter
aufweist, wobei das in Abb. 1 wiedergegebene IR-KBr-
Absorptionsspektrum von besonderer Bedeutung ist.

Von diesen ist der neue Mikroorganismenstamm BA 9
der Gattung Streptomyces im Rahmen der vorliegenden
Erfindung besonders wichtig.

Le A 23 433

Die Anreicherung und Isolierung des Stammes erfolgte
nach den üblichen Methoden der Actinomyceten-Isolierung
durch Ausplattieren von Erdprobensuspensionen auf Petrischalen, vier- bis sechswöchige Bebrütung und wiederholter Abimpfung von Einzelkolonien (Williams, S.T. and
Cross, T. (1971). Actinomycetes. In Methods in Microbiology 4, 295-334. Booth, C. (editor), London-New York:
Academic Press).

Der neue Streptomyces-Stamm mit den Laborbezeichnung
BA 9 wurde am 06. August 1984 unter der Nummer
DSM 3025 bei der Deutschen Sammlung von Mikroorganismen, Griesebachstraße 8, 3400 Göttingen, BRD hinterlegt.

Es wurde weiterhin gefunden, daß man die erfindungsgemäße Verbindung erhält, wenn man geeignete Mikroorganismen der Familie Streptomycetaceae in einem
Nährmedium, welches assimilierbare Kohlenstoff-
und Stickstoffquellen sowie Mineralsalze enthält,
unter aeroben Bedingungen kultiviert und die Verbindung nach üblichen Methoden isoliert.

Zur Durchführung des erfindungsgemäßen Verfahrens
kann insbesondere der Streptomyceten-Stamm BA 9
(sowie seine Mutanten und Varianten) Verwendung
finden.

Dieser Stamm gehört zu den Bakterien der Ordnung Actinomycetales, der Familie Streptomycetaceae und der Gattung
Streptomyces an. Er wurde aus Erde isoliert.

Le A 23 433

Die taxonomische Bestimmung des Stammes BA 9 erfolgte nach Bergey's Manual of Determinative Bacteriology

8th ed. (1974) sowie International Journal of Systematic Bacteriology 16, 313-340 (1966) und The Prokaryotes 2, 2028-2090 (1981).

1. Morphologie

Eine gute Sporulation war nur auf inorganic salts starch-Agar (ISP-Medium Nr. 4 Zusammensetzung siehe S. 7) zu beobachten. Auf anderen Medien (ISP-Medien Nr. 1[+]-3 und 5) wurde wenig Luftmyzel bzw. nur Substratmyzel gebildet.

[+] ISP-Medium Nr. 1, supplementiert mit 10 g/l D-Mannit (Zusammensetzung siehe S. 7).

Luftmyzel (ISP-Medium Nr. 4; 28°C; 7 d):

Farbe: Nach 7d grau
Sporenketten: Spira-Typ
Sporen: leicht rauh (Elektrononmikroskopie),
         zylindrisch ca. 1,1-1 µm breit, 1,4-1,6
         µm lang

Substratmyzel:

Farbe: rotbraun, insbesondere auf ISP-Medium Nr.
       3 und 5

Le A 23 433

Zellwandanalyse:

Zellwandtyp I, Hauptbestandteile sind LL-Diaminopimelinsäure und Glycin.

2. Physiolgie

Das Temperaturoptimum liegt bei 28° (ISP-Medium Nr. 1$^+$, 5 d). Auf ISP-Medium Nr. 7 (Tyrosin-Agar) wurde kein Wachstum beobachtet. Melanin wird auf ISP-Medium Nr. 6 gebildet. Das Wachstum wird durch Chloramphenicol (10 µg); Erythromycin (10 µg), Sulphafurazol (100 µg), Streptomycin (10 µg), Tetracyclin (10 µg), Chephaloridin (5 µg) Fusidinsäure (10 µg), Lincomycin (10 µg), Novobiocin (5 µg) gehemmt (ISP-Medium Nr. 1$^+$, 28°C, 2d). Gutes Wachstum (Substratmyzel) erfolgt auf den ISP-Medien Nr. 3,4,5 mit spärlicher Luftmyzelbildung auf ISP-Medium Nr. 3 und 5.

ISP-Medium Nr. 4 (inorganic salts-starch agar):

| | |
|---|---|
| Stärke (löslich) | 10,0 g |
| $K_2HPO_4$ (anhydr.) | 1,0 g |
| $MgSO_4$ 7 $H_2O$ | 1,0 g |
| NaCl | 1,0 g |
| $(NH_4)_2SO_4$ | 2,0 g |
| $CaCO_3$ | 2,0 g |
| Spurenelementlösung | 1,0 ml |
| $H_2O$ deionisiert | 1000,0 ml |

pH 7,0 - 7,4


Spurenelementlösung

| | |
|---|---|
| $FeSO_4$ 7 $H_2O$ | 0,1 g |
| $MnCl_2$ 4 $H_2O$ | 0,1 g |
| $ZnSO_4$ 7 $H_2O$ | 0,1 g |
| $H_2O$ deion. | 100,0 ml |


ISP-Medien Nr. 1 (Trypton-Hefeextrakt-Agar) supplementiert mit Mannit:

| | |
|---|---|
| Bacto-Trypton (Difco) | 5,0 g |
| Bacto-Hefeextrakt (Difco) | 3,0 g |
| Bacto-Agar (Difco) | 15,0 g |
| D-Mannit | 10,0 g |
| $H_2O$ deion. | 1000,0 ml |

Weitere Medien s. Int. J. Syst. Bact. **16**, 313-340 (1966).


Le A 23 433

Test auf Verwertung von Kohlenstoff (C)-Quellen:

Die Verwertung von C-Quellen wurde auf Basalager nach Int. J. Syst. Bact. <u>16</u>, 313-340 (1966) überprüft. Für die Negativ-Kontrolle wurde das Wachstum auf Basalager ohne C-Quelle verglichen.

<u>Le A 23 433</u>

| C-Quelle (10 g/l) | Wachstum[*] |
|---|---|
| Kontrolle (keine C-Quelle) | - |
| D-Glucose | + |
| D-Xylose | - |
| L-Arabinose | - |
| L-Rhamnose | + |
| D-Fructose | + |
| D-Galactose | + |
| Raffinose | - |
| D-Mannit | + |
| Meso-Inosit | - |
| Salicin | - |
| Saccharose | - |
| Ribose | - |
| Mannose | - |
| Maltose | - |
| Lactose | ± |
| Mellibiose | - |
| Cellulose | - |
| Acetat | - |

*)　+ = Wachstum;

　　- = kein Wachstum;

　　± = Ergebnis nicht eindeutig.

Le A 23 433

Der Stamm BA 9, isoliert aus einer Erdprobe aus Israel
(Jerusalem) läßt sich aufgrund der morphologischen
Daten in die graue Serie der Streptomyceten (Cinereus-
Gruppe) einordnen. Die physiologischen Eigenschaften
stimmen mit keinem der in Bergey's Manual beschriebenen Stämme völlig überein.

Taxonomische Bezeichnung: Streptomyces sp.

Für das Verfahren zur Herstellung des erfindungsgemäßen Verbindung verwendet man Nährmedien, die die
üblichen Kohlenstoff- und Stickstoffquellen und die
notwendigen Salze enthalten. Als Kohlenstoffquelle
können verwendet werden:

Kohlenhydrate, insbesondere Polysaccharide, wie z.B.
Stärke, Monosaccharide, wie z.B. Glucose oder Fructose.
Weiterhin können noch verwendet werden Zuckeralkohole,
wie z.B. Mannit oder Glycerin, außerden auch natürlich
vorkommende Gemische, wie z.B. Malzextrakt oder Distiller soluble sowie Gemische aus den erwähnten Möglichkeiten.

Als Stickstoffquelle können die üblichen Stickstoffquellen Verwendung finden, so z.B. Eiweißstoffe, Eiweißhydrolysate, Aminosäuren, Ammoniumsalze, natürlich
vorkommende komplexe Stoffe wie Sojabohnenmehl,
Fleischextrakte, Hefeextrakte, Milchpulver und geeignete Mischungen derselben.

Le A 23 433

Als Hilfsstoffe werden im Nährmedium Mineralsalze benötigt, z.B. Phosphate, Sulfate oder Chloride, des Kaliums, des Natriums, des Calciums, des Magnesiums, Eisens, Zinks und Mangans. Die Konzentration dieser Stoffe kann in weiten Grenzen schwanken, z.T. sind die notwendigen Konzentrationen der Mineralsalze in den o.a. Kohlenstoff- oder Stickstoffquellen oder im verwendeten Wasser als Verunreinigungen enthalten.

Weiterhin können als Hilfsstoffe noch Antischaummittel der verschiedensten Art Verwendung finden, wie z.B. Polyole oder Silikone.

Das Verfahren zur Herstellung der erfindungsgemäßen Verbindung kann mit Hilfe üblicher fester, halbfester oder flüssiger Nährmedien durchgeführt werden. Bevorzugt werden wäßrig flüssige Nährmedien.

Die Beimpfung der Nährmedien erfolgt dabei nach allgemein üblichen Methoden, z.B. über Schrägröhrchen oder Kolbenkulturen.

Die Kultur erfolgt unter aeroben Bedingungen und kann gemäß den allgemein üblichen Methoden wie unter Verwendung von Schüttelkulturen z.B. in Schüttelkolben, von luftbewegten Kulturen oder von Submerskulturen durchgeführt werden. Bevorzugt erfolgt die Kultivierung im aeroben Submersverfahren in belüfteten Fermentern, z.B. in üblichen Submersfermentationstanks. Es ist möglich, die Kultur kontinuierlich oder diskontinuierlich durchzuführen. Vorzugsweise wird diskontinuierlich gearbeitet.

Le A 23 433

Bei der Durchführung des Herstellungsverfahrens wird unter aeroben Bedingungen gearbeitet; die Kultur kann gemäß üblicher Methoden, so z.B. unter Verwendung von Schüttelkulturen oder von belüfteten Fermenterkulturen, durchgeführt werden. Die Prozentverhältnisse der Nährlösungsbestandteile können in weiten Bereichen schwanken, im allgemeinen machen die Kohlenstoffquellen 0,5 - 8 %, vorzugsweise 0,6 - 6%, aus, die Stickstoffquellen 0,1 - 5 %, vorzugsweise 0,5 - 2 %, aus, die Salze liegen in üblichen Konzentrationen vor, vorzugsweise im Bereich zwischen 0,001 - 0,5 Gew.-%. Die Antischaummittel liegen in 0,5 %iger Konzentration vor. Die zur Sterilisation angewandten Temperaturen liegen bei 100 - 140°C, bevorzugt bei 120 - 130°C.

Der pH-Wert der wachsenden Kulturen sollte vorzugsweise zwischen etwa 6 und etwa 8,5, insbesondere zwischen 6,5 und 8,0 gehalten werden. Ein zu starker pH-Abfall in den sauren Bereich kann durch Zusätze einer organischen oder anorganischen Base, vorzugsweise von $CaCO_3$ vermieden werden. Wie in der Fermentationstechnologie üblich, kann auch eine automatische pH-Regulierung durchgeführt werden, bei der sterile organische oder anorganische Säure, z.B. $H_2SO_4$, oder sterile Lauge, z.B. NaOH in Abständen in die Kulturlösung eingespritzt wird.

Es ist zweckmäßig sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Le A 23 433

Die Züchtungstemperatur kann zwischen etwa 16 und etwa 42°C, vorzugsweise zwischen 24 und 32 °C liegen, besonders bevorzugt liegt sie bei etwa 28 °C. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden erfindungsgemäßen Verbindung im allgemeinen ihr Maximum etwa 1 bis 7, vorzugsweise 1 bis 4 Tage nach Züchtungsbeginn erreicht.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefässe sowie der für die Belüftung notwendigen Luft. Zur Sterilisation der Vorrichtungen können z.B. die Dampf- als auch die Trockensterilisation verwendet werden.

Falls bei der Kultivierung in unerwünschter Menge Schaum entsteht, können in üblichen chemischen Schaumdämpfungsmittel, z.B. flüssige Fette und Öle, Öl-Wasser-Emulsionen, Raraffine, höhere Alkohole, wie Octodecanol, Siliconöle, Polyoxyäthylen- bzw. Polyoxypropylenverbindungen zugesetzt werden. Schaum kann auch mit Hilfe der üblichen mechanischen Vorrichtungen (welche z.B. Zentrifugalkräfte benutzen) gedämpft oder beseitigt werden.

Le A 23 433

Die erfindungsgemäße Verbindung kann aus dem Kulturmedium nach allgemein üblichen physikalisch-chemischen Methoden isoliert werden. Die Isolierung kann z.B. gemäß den üblichen Extraktionsverfahren, Fällungsverfahren und/oder Chromatographieverfahren erfolgen. Die isolierte Verbindung kann mit Hilfe der genannten Methoden auch feingereinigt werden. Für viele Fälle ist jedoch eine Feinreinigung nicht erforderlich, da die gegebenenfalls vorhandenen Verunreinigungen die Wirksamkeit der Verbindung nicht nachteilig beeinflussen.

Um bei den o.a. Isolierungs- und Reinigungsmethoden die Fraktionen herauszufinden, in welchen die erfindungsgemäße Verbindung in höchster Konzentration bzw. Reinheit vorliegt, wird bevorzugt die Bestimmung der antibiotischen Aktivität herangezogen werden. Als Testkeime eignen sich dabei besonders Staphylococcus aureus 1756, Bac. subtilis ATCC 6633 und Escherichia Coli 14.

Die Isolierung und Reinigung der erfindungsgemäßen Verbindung kann z.B. im Falle, daß ein flüssiges wäßriges Nährmedium verwendet wird, wie folgt vorgenommen werden: Nach seiner Anreicherung im Kulturüberstand werden Kulturfiltrat und Mycel mit üblichen Methoden separiert (z.B. Zentrifugation).

Aus dem Kulturfiltrat kann die erfindungsgemäße Verbindung mit Hilfe von üblichen Extraktionsverfahren, Fällungsverfahren und/oder Chromatographieverfahren isoliert und

Le A 23 433

gegebenenfalls gereinigt werden. Die Chromatographie kann in Form der Säulenchromatographie durchgeführt werden. Als Adsorptionsmittel können die üblichen anorganischen oder organischen Adsorptionsmittel eingesetzt werden, wie z.B. Aluminiumoxid, Kieselgel, Magnesiumsilikat, Aktivkohle, Cellulose, Cellulosederivate, Kunstharze wie Polyamiden, z.B. acetyliertes Polyamid oder Dextrangele. Als Laufmittel können die verschiedensten Lösungsmittel oder Lösungsmittelgemische verwendet werden, in welchen die erfindungsgemäße Verbindung löslich ist. Bevorzugt wird Methanol oder ein Gemisch aus Chloroform und Methanol (z.B. 1:1 Volumenteile) eingesetzt.

Bevorzugt werden zur Isolierung der erfindungsgemäßen Verbindung Chromatographie-Verfahren verwendet, z.B. unspezifische Adsorption an hydrophobe Sorbentien oder andererseits Geldiffusionschromatographie. Die sind die von der Reinigung schlecht wasserlöslicher Naturstoffe her bekannte Methoden.

Für die kommerzielle Herstellung der erfindungsgemäßen Verbindung bevorzugt man die Gewinnung durch Adsorption und anschließende Desorption an einem hydrophoben Trägerharz (z.B. Lewapol; ein hydrophobes Trägerharz der BAYER AG). Die Desorption kann z.B. mit kurzkettigen aliphatischen Alkoholen durchgeführt werden, bevorzugt Methanol oder Ethanol.

Le A 23 433

Eine derart vorgereinigte Fraktion kann wiederung mit den üblichen Methoden gereinigt werden. Bevorzugt kann hier die Geldiffusionschromatographie eingesetzt werden. Die Chromatographie an Sephadex LH-20 $^{®}$ verläuft erfolgreich. Ein derartig gewonnenes Produkt ist im allgemeinen reiner als 50 %.

Bevorzugt wird zur Isolierung und Reinigung der erfindungsgemäßen Verbindung wie folgt gearbeitet:

Das Mycelium wird aus der Kulturbrühe, vorzugsweise durch Zentrifugation abgetrennt und mit einem, mit Wasser mischbaren Lösungsmittel mehrfach, vorzugsweise zweimal extrahiert. Als Lösungsmittel können niedere Alkylalkohole ($C_1$-$C_4$) wie Ethanol oder Isopropanol sowie Ketone wobei besonders bevorzugt Aceton ist, verwendet werden. Die wäßrig-organische Lösung wird im Vakuum, z.B. etwa auf 1/20 des Volumens der Kulturbrühe konzentriert und gefriergetrocknet.

Dieses Rohpräparat wird mit Wasser suspendiert und die erfindungsgemäße Verbindung mit einem mit Wasser nicht mischbaren Lösungsmittel, wie z.B. Ester der Essigsäure oder Ketone extrahiert. Aus diesem Extrakt kann die Verbindung durch übliche chromatographische Methoden, vorzugsweise Chromatographie an Sephadex LH 20 $^{(R)}$ und an Kieselgel isoliert werden.

Le A 23 433

Die Verbindung kann auch aus dem Kulturfiltrat durch Adsorption an Aktivkohle bzw. an geeignete Harze isoliert werden. Als besonders geeignete Methode erwies sich die Bindung der erfindungsgemäßen Verbindung an unspezifische Adsorberharze auf Polystyrolbasis (z.B. Amberlite XAD der Fa. Roehm & Haas oder Lewatit OC 1031 der Fa. Bayer). Die Desorption der erfindungsgemäßen Verbindung wird fraktioniert, durch Mischungen aus Wasser und organischen Lösungsmitteln, isbesondere Wasser/Methanol vorgenommen. Die durch Test gegen Staphylococcus aureus 1756 ermittelten aktiven Fraktionen werden unter reduziertem Druck bis zur vollständigen Entfernung des organischen Lösungsmittels konzentriert und den Rückstand in ca. 1/100 des Volumens des Kulturfiltrates suspendiert und gefriergetrocknet.

Das Lyophilisat wird nun mit Wasser suspendiert und vorzugsweise mit Essigsäureethylester oder anderen nicht mit Wasser mischbaren Lösungsmitteln extrahiert. Aus dem Extrakt kann die erfindungsgemäße Verbindung durch übliche chromatographische Methoden, vorzugsweise Chromatographie an Sephadex LH 20 (R) und Kieselgel gewonnen werden.

Wie bereits erwähnt wirkt die erfindungsgemäße Verbindung antibakteriell. Dies kann wie folgt gezeigt werden:

Le A 23 433

Von verschiedenen Testkeimen werden Übernachtkulturen hergestellt: Mit je 1 ml dieser Ansätze wird sterilisiertes, vortemperiertes Medium angeimpft. Dann werden die Testkeime bei geeigneter Temperatur auf der rotierenden Schüttelmaschine solange inkubiert, bis die Zellen die log-Phase erreicht haben. Diese werden dann mit sterilem, vortemperiertem Nährmedium bis zu einer $OD_{578}$ (OD = Optical Densitiy = scheinbare Extinktion) von 0,02 - 0,04 herunterverdünnt. Diesen Suspensionen wird der Wirkstoff zugegeben, und mittels Verdünnungsreihen werden verschiedene Wirkstoffkonzentrationen eingestellt. Wenn die Kontrollansätze (ohne Wirkstoff) nach geeigneter Inkubation eine starke Trübung zeigen, wird die MHK (MHK = Minimale Hemmkonzentration) ermittelt, die der niedrigsten Wirkstoffkonzentration, bei denen die Ansätze keine Trübung zeigen, entspricht.

Die Versuche wurden mit Antibiotika Medium 3 durchgeführt. Inkubationszeit bis zur Messung, Ausgangs-OD und Inkubationstemperatur der verschiedenen Testkeime sind der folgenden Tabelle 1 zu entnehmen:

Le A 23 433

Tabelle 1

| Testkeim | MHK µg/ml | Inkubationstemperatur °C | Inkubationszeit bis zur Messung | $OD_{578}$ |
|---|---|---|---|---|
| Bacillus brevis | 2,5 | 37 | 6 h | 0,02 |
| Bacilllus cereus | 0,6 | 37 | 5 h | 0,04 |
| Bacillus subtilis ATCC 6633 | 0,6 | 37 | 4,5 h | 0,02 |
| Ecoli 14 | 1,3 | 37 | 4 h | 0,02 |
| Ecoli A 261 | 2,5 | 37 | 4 h | 0,02 |
| Proteus vulgaris | 1,3 | 37 | 4 h | 0,04 |
| Pseudomonas aeruginosa | 10 | 27 | 4 h | 0,02 |
| Pseudomnas aeruginosa Ellsworth | 10 | 37 | 4 h | 0,03 |
| Serratia mareescens | 2,5 | 30 | 4,5 h | 0,02 |
| Staphylococcus aureus A 1756 | 1,3 | 37 | 4 h | 0,02 |
| Staphylococcus aureus P 209 | 0,6 | 37 | 4 h | 0,02 |

Die erfindungsgemäße Verbindung zeigt eine gute antibakterielle Wirkung gegen verschiedene grampositive
und gramnegative Keime:

Sie läßt sich sowohl in der Human- als auch in der Tiermedizin als Therapeutikum einsetzen. Besonders erwähnt
werden soll ihre Eignung zur Verbindung und Heilung
von Schweinedysenturie und von Ketose bei Rindern.

Die erfindungsgemäße Verbindung läßt sich in antibakteriell wirksamen Zubereitungen verwenden. Besonders
bevorzugt sind solche antibakteriell wirksamen Zubereitungen, die die erfindungsgemäße Verbindung oder
deren Alkali- oder Erdalkalisalze enthalten.

Die erfindungsgemäßen pharmazeutischen Zubereitungen
enthaltnen neben den neuen erfindungsgemäßen Verbindungen
in üblicher Weise nicht toxische, inerte pharmezeutisch
geeignete Trägerstoffe. Solche pharmazeutisch geeignete
Trägerstoffe sind beispielsweise Füll- und Streckmittel,
Bindemittel, Feuchthaltemittel, Lösungsverzögerer,
Resorptionsbeschleuniger, Netzmittel, Adsorbtionsmittel
oder Gleitmittel, die feste, halbfeste oder flüssige
Konsistenz haben können. Solche pharmazeutisch geeignete
Trägerstoffe sind dem Fachmann bekannt.

Le A 23 433

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotionen, Puder und Sprays genannt. Die Herstellung dieser Zubereitungen geschieht nach bekannten Methoden in üblicher Weise, beispielsweise durch Mischen der neuen erfindungsgemäßen Verbindung mit den üblichen Träger- und Zusatzstoffen. Der Wirkstoff soll in den aufgeführten pharmazeutischen Zubereitungen in einer Konzentration von etwa 0,1 bis 99,5, vorzugsweise von etwa 0,5 bis 95 Gew.-% der Gesamtmischung vorhanden sein.

Die erfindungsgemäße Verbindung kann in allen Bereichen der Tierzucht als Mittel zur Förderung und Beschleunigung des Wachstums und zur Verbesserung der Futterverwertung bei gesunden und kranken Tieren verwendet werden.

Die Wirksamkeit des Wirkstoffs ist hierbei weitgehend unabhängig von der Art und dem Geschlecht der Tiere. Besonders wertvoll erweist sich der Wirkstoffebei der Aufzucht und Haltung von Jung- und Masttieren.

Als Tiere, bei denen der Wirkstoff zur Förderung und Beschleunigung des Wachstums, zur Verbesserung der Futterverwertung sowie zur Verbesserung des Fleisch/Fett-Verhältnisses in Schlachtkörper eingesetzt werden können, seien beispielsweise folgende Nutz- und Ziertiere genannt:

Le A 23 433

Warmblüter wie Rinder, Schweine, Pferde, Schafe, Ziegen, Katzen, Hunde, Kaninchen, Pelztiere, z.B. Nerze, und Chinchilla, Geflügel, z.B. Hühner, Gänse, Enten, Truthähne, Tauben, Papageien und Kanarienvögel und Kaltblüter wie Fische, z.B. Karpfen und Reptilien, z.B. Schlangen.

Die Mengen des Wirkstoffs, die den Tieren zur Erreichung des gewünschten Effektes verabreicht wird, kann wegen der günstigen Eigenschaften des Wirkstoffs weitgehend variiert werden. Sie liegt vorzugsweise bei etwa 0,005 bis 50, insbesondere 0,1 bis 10 mg/kg Körpergewicht pro Tag. Die Dauer der Verabreichung kann von wenigen Stunden oder Tagen bis zu mehreren Jahren betragen. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Der Wirkstoff wird den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen.

Le A 23 433

Aus Zweckmäßigkeitsgründen ist in den meisten Fällen eine orale Verabreichung, insbesondere im Rhythmus der Nahrungs- und/oder Getränkeaufnahme der Tiere, vorzuziehen.

Der Wirkstoff kann als reine Stoffmischung oder in formulierter Form, also in Mischung mit nicht-toxischen inerten Trägerstoffen beliebiger Art, z.B. mit Trägerstoffen und in Formulierungen, wie sie bei nutritiven Zubereitungen üblich sind, verabreicht werden.

Der Wirkstoff wird gegebenenfalls in formulierter Form zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht.

Empfehlenswert ist die orale Verabreichung zusammen mit dem Futter und/oder Trinkwasser, wobei je nach Bedarf der Wirkstoff der Gesamtmenge oder nur Teilen des Futters und/oder des Trinkwassers zugegeben wird.

Der Wirkstoff wird nach üblichen Methoden durch einfaches Mischen als reine Stoffmischung, vorzugsweise in fein verteilter Form oder in formulierter Form in Mischung mit eßbaren nicht-toxischen Trägerstoffen, gegebenenfalls in Form eines Praemix oder eines Futterkonzentrates, dem Futter und/oder Trinkwasser beigefügt.

Das Futter und/oder Trinkwasser kann beispielsweise den Wirkstoff in einer Gewichtskonzentration von etwa

Le A 23 433

0,005 bis 50, insbesondere 0,1 bis 10 ppm, enthalten.
Die optimale Höhe der Konzentration des Wirkstoffs in
dem Futter und/oder Trinkwasser ist insbesondere abhängig von der Menge der Futter- und/oder Trinkwasseraufnahme der Tiere und kann durch jeden Fachmann leicht
ermittelt werden.

Die Art des Futters und seine Zusammensetzung ist
hierbei ohne Belang. Es können alle gebräuchlichen
oder speziellen Futterzusammensetzungen verwendet
werden, die vorzugsweise das übliche, für eine ausgewogene Ernährung notwendige Gleichgewicht aus
Energie- und Aufbaustoffen einschließlich Vitaminen
und Mineralstoffen enthalten. Das Futter kann sich
beispielsweise zusammensetzen aus pflanzlichen
Stoffen, z.B. Heu, Rüben, Getreide, Getreidenebenprodukten, tierischen Stoffen, z.B. Fleisch, Fetten,
Knochenmehl, Fischprodukten, Vitaminen, z.B. Vitamin A,
D-Komplex und B-Komplex, Proteinen, Aminosäuren, z.B.
DL-Methionin und anorganischen Stoffen, z.B. Kalk
und Kochsalz.

Futterkonzentrate enthalten den Wirkstoff neben eßbaren Stoffen, z.B. Roggenmehl, Maismehl, Sojabohnenmehl oder Kalk, gegebenenfalls mit weiteren Nähr- und
Aufbaustoffen sowie Proteinen, Mineralsalzen und Vitaminen. Sie können nach den üblichen Mischmethoden
hergestellt werden.

Le A 23 433

Vorzugsweise in Praemixen und Futterkonzentraten kann der Wirkstoff gegebenenfalls auch durch ihre Oberfläche bedeckende geeignete Mittel, z.B. mit nicht-toxischen Wachsen oder Gelatine vor Luft, Licht und/oder Feuchtigkeit geschützt werden.

Beispiel für die Zusammensetzung eines Kükenaufzuchtfutters, das den erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

In einem kg Futtermischung sind enthalten: 600 I.E. Vitamin A, 100 I.E. Vitamin D, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$.

Der Wirkstoff-Praemix enthält den Wirkstoff in der gewünschten Menge, z.B. 10 mg und zusätzlich 1 g DL-Methionin sowie so viel Sojabohnenmehl, daß 2,5 g Praemix entstehen.

Le A 23 433

Beispiel für die Zusammensetzung eines Schweineaufzuchtfutters, das den erfindungsgemäßen Wirkstoff
enthält:

630 g Futtergetreideschrot (zusammengesetzt aus
200 g Mais, 150 g Gerste-, 150 g Hafer- und
130 g Weizenschrot), 80 g Fischmehl, 60 g Sojaschrot, 60 g Tapiokamehl, 38 g Bierhefe, 50 g
Vitamin-Mineral-Mischung für Schweine (Zusammensetzung z.B. wie beim Kükenfutter), 30 g Leinkuchenmehl, 30 g Maiskleberfutter, 10 g Sojaöl, 10 g
Zuckerrohrmelasse und 2 g Wirkstoff-Praemix (Zusammensetzung z.B. wie beim Kükenfutter) ergeben
nach sorgfältigem Mischen 1 kg Futter.

Die angegebenen Futtergemische sind vorzugsweise zur
Aufzucht und Mast von Küken bzw. Schweinen abgestimmt,
sie können jedoch in gleicher oder ähnlicher Zusammensetzung auf zur Aufzucht und Mast anderer Tiere verwendet werden.

Wie bereits erwähnt ist es nicht unbedingt erforderlich
den gereinigten und isolierten Wirkstoff zu verwenden.
Es ist auch möglich die bei seiner Herstellung anfallende Kulturbrühe ohne Reinigung, gegebenenfalls
nach Trocknung, einzusetzen. Für viele Zwecke ist es
auch ausreichend Rohformen der erfindungsgemäßen Wirkstoffe ohne vorherige Feinreinigung einzusetzen.

Die Herstellung der neuen erfindungsgemäßen Verbindung
kann durch die folgenden Beispiele erläutert werden:

Le A 23 433

## Beispiel 1

Die Nährlösung, in der der Produktionsstamm Streptomyces sp. BA 9 kultiviert wird, setzt sich aus Sojamehl, entfettet, 3 % Glycerin (87 %ig) 3 %, $CaCO_3$ 0,2 % in Leitungswasser zusammen. Die Sterilisation der Nährlösung wird 40 min bei 121°C durchgeführt. Ein 1000 ml Erlenmeyer-Kolben, der 150 ml dieser Nährlösung enthält, wird mit dem Produktionsstamm beimpft und 5 Tage bei 28°C auf der rotierenden Schüttelmaschine bei 280 Umdrehungen/min inkubiert. Mit der so erhaltenen Vorkultur wird ein 40 l-Fermenter, der 20 l der oben beschriebenen Nährlösung, zusätzlich 0,05 % Silikon, enthält, beimpft und bei 200 Umdrehungen/min (Blattrührer), 10 l Luft/min, 1,0 bar Überlagerungsprodukt und 28°C 2 Tage inkubiert. Mit diesem Vorfermenter wird der 600 l Produktionsfermenter beimpft, der 400 l obiger Nährlösung, zusätzlich 0,03 % Silikon, enthält. Die Inkubation der Produktionskultur erfolgt 65 h bei 28°C, einem Überlagerungsdruck von 1,0 bar, einer Rührung von 60 Umdrehungen/min (Blattrührer) und einer Belüftung von 160 l Luft/min.

Le A 23 433

## Beispiel 2

Die gemäß Beispiel 1 erhaltene Kulturbrühe einer 400 l-Fermentation wird mit 200-250 l/h in einem Westfalia-Separator separiert. Der Überstand wird über eine mit 50 l Lewatit OC 1031 (Bayer) beschickte Säule von 30 cm Durchmesser gegeben. Nacheinander wird mit 500 l entmineralisiertem Wasser, 300 l 30 %igem wäßrigen Methanol gewaschen und mit 300 l Methanol desorbiert. Das Desorbat wird unter reduziertem Druck konzentriert bis zur Trockne und in 20 l Wasser suspendiert und gefriergetrocknet, wobei 137 g der rohen erfindungsgemäßen Verbindung erhalten wurden (Wirkstoffgehalt 1,5 %).

## Beispiel 3

Reindarstellung der erfindungsgemäßen Verbindung durch Gelfiltration und Kieselgelchromatographie.

50 g des nach Beispiel 2 erhaltenen Rohproduktes wurden in 2 l Wasser suspendiert und der pH-Wert mit Essigsäure konz. auf 5,0 eingestellt. Diese Suspension wird zweimal mit je 2 l eines Gemisches von Ethylacetat/Ethanol im Volumenverhältnis 9:1 im Scheidetrichter extrahiert. Die vereinigten organischen Phasen werden unter reduziertem Druck konzentriert bis zur Trockne in 100 ml Methanol gelöst und einer Gelfiltration auf Sephadex LH 20 (R)

Le A 23 433

in Methanol unterworfen. Es wird eine Säule und Durchmesser 7,5 cm und von 1,25 m Länge verwendet. Die Fließrate beträgt 720 ml/h. 200 ml-Fraktionen werden gesammelt. Die nach Antibiographie gegen Staphylococcus areus 1756 wirksamen Fraktionen werden vereinigt, unter reduziertem Druck konzentriert, in Wasser suspendiert und gefriergetrocknet.

Ausbeute: 2,94 g eines 26 %igen Produktes.

600 mg des so erhaltenen Materials werden in 100 ml Chloroform unter Zusatz von 60 µl Essigsäure konz. gelöst. Eine Lobar[(R)]-Fertigsäule LiChroprep 60 (40-63 µm, Merck, Typ B) wird mit Chloroform unter Zusatz von 0,1 % Essigsäure konz. (v/v) äquilibriert und die Lösung aufgetragen. Nach Elution mit 400 ml des obigen Fließmittels wird die Substanz mit einem Gemisch von Chloroform/Methanol/Essigsäure 98/2/0,1 eluiert. Die Fließrate beträgt 400 ml/h. 10 ml-Fraktionen werden gesammelt.

Sie werden dünnschichtchromatographisch auf Kieselgelplatten (Merck 5729) analysiert. Die biologisch aktiven, mit $FeCl_3/H_2SO_4$ anfärbbaren, einheitlichen Fraktionen werden konzentriert bis zur Trockne, mit wenig Wasser suspendiert und gefriergetrocknet.

Ausbeute: 76,2 mg eines 98 %igen Materials.

<u>Le A 23 433</u>

Beispiel A:

Einem Hammel, der pro Tag 650 g grob gemahlenes Schaf-Fertigfutter und 250 g Trockengrün Cobs erhielt, wurde Pansenflüssigkeit durch eine Pansenfistel entnommen. Das Fertigfutter wurde über einen Futterautomaten in 12 gleiche Portionen in zweistündlichem Abstand und die Cobs in 2 gleichen Portionen um 8.30 und 16.15 Uhr verabreicht. Die Pansenflüssigkeit wurde unmittelbar nach der Gewinnung folgender Prozedur unterworfen: 2,5 ml des Panseninokulums wurde in einem 13 ml mit Kohlendioxid begasten Reagenzröhrchen vorgelegt, das darüber hinaus folgende Zusätze enthielt:

100 mg fein gemahlenes Schaft-Fertigfutter

7,5 ml Pufferlösung

0,5 ml einer Lösung mit bzw. ohne erfindungsgemäße Verbindung.

Die Zusammensetzung der Pufferlösung, welche vor dem Anfang des Versuches mit Kohlendioxid gesättigt wurde, war wie folgt:

| | | |
|---|---|---|
| $Na_2HPO_4$ | 4,61 g | per Liter Wasser |
| $NaHCO_3$ | 12,25 g | "          "          " |
| NaCl | 0,59 g | "          "          " |
| KCl | 0,71 g | "          "          " |
| $MgCl_2$ | 0,32 g | "          "          " |
| $CaCl_2$ | 0,13 g | "          "          " |

Le A 23 433

Die erforderliche Menge der erfindungsgemäßen Verbindung um 10, 20, 30 oder 40 µg pro Ansatz zu erhalten, wurde in 0,25 ml Ethanol gelöst, danach erfolgte die Zugabe von 4,75 ml Wasser. Aus dieser Lösung wurden 0,5 ml in ein Reagenzglas pipettiert. Danach betrug das totale Volumen der Fermentationsmischung 10,5 ml. Die negativen Kontrollen (d.h. Ansätze ohne Testsubstanz) erhielten ebenfalls 0,5 ml einer 5 %igen Ethanol-Lösung. Es wurden 4 Wiederholungen pro Dosierung durchgeführt. Jedes Reagenzröhrchen wurde mit einem Bunsen-Stopfen verschlossen und bei 39°C bebrütet. Nach 2, 4, 6 und 8 Stunden wurden die Ansätze per Hand geschüttelt. Nach 24-stündiger Bebrütung wurde 1,0 ml der Fermentationsflüssigkeit aus den Ansätzen entnommen und in einem Eppendorf-Gefäß pipettiert, das 0,2 ml 10 %ige Phosphorsäure (enthaltend 5,7 µmol 2-Methylvaleriansäure) enthielt, getan. Die Proben wurden bei 10 000 g zentrifugiert und aus dem Überstand die flüchtigen Fettsäurekonzentrationen gaschromatographisch bestimmt.

Die Analysenergebnisse sind in der nachstehenden Tabelle a zusammengestellt. Die Daten (das Essigsäure/Propionsäure-Verhältnis und die Gesamtfettsäurenproduktion) sind als Prozent der Negativ-Kontrolle dargestellt:

Le A 23 433

Tabelle a

| Behandlung (µg/Ansatz) | Essigsäure- Propionsäure- Verhältnis (%) | Gesamtfettsäuren (%) |
|---|---|---|
| 10 | ⊦ 99,7 | 103,3 ⊦ |
| 20 | 75,5 | 107,9 |
| 30 | 78,8 | 96,7 |
| 40 | 75,9 | 96,0 |

Die Tabelle a zeigt, daß das Verhältnis von Acetat zu Propionat wesentlich herabgesetzt und damit verbessert wird; durch die vermehrte Bildung von Propionat aus den Kohlehydraten kommt es zu einer Verbesserung der Futterverwertung. Die erhöhte Effizienz der Gärung geht nur in geringem Maße auf Kosten der Gesamtfettsäureprodukten, wie ebenfalls aus der Tabelle a ersichtlich ist.

Beispiel B:

Masthähnchen (Broiler):

Die in Käfigen gehaltenen Tiere werden im Alter von 3-5 Tagen für den Versuch herangezogen, der sich insgesamt über einen Zeitraum von 14 Tagen erstreckt.

Le A 23 433

Die Tiere erhalten während dieser Zeit Futter, welchem die erfindungsgemäße Verbindung in einer Dosis von 10 bzw. 25 ppm beigemischt wird. Eine Negativkontrolle (unsupplementiertes Futter) wird mitgeführt. Bei Versuchsbeginn haben alle Tiere einer Versuchsgruppe das gleiche Ausgangsgewicht.

Als Beurteilungskriterien werden die Gewichtszuwachsraten, der Futterverbrauch und die Futterverwertung herangezogen.

Die Ergebnisse sind in der nachstehenden Tabelle b dargestellt.

Tier-Charakteristik und Futter

Sortierte Masthybriden, männlich
24 Tiere/Versuch (4x6)
Gewicht 50 - 65 g
Futter: Höveler Kükenalleinfutter KA 57 ohne Zusätze
        von Antibiotika und Coccidiostatika folgender
        Zusammensetzung:

Wertbestimmende Bestandteile:

18    % Rohprotein
 7    % Rohfaser
 8    % Asche
 1    % Kalzium
0,7   % Phosphor

Le A 23 433

Zusammensetzung:

54 % Futtergetreideschrot (40 % Mais, 12 % Weizen)
17,5 % Sojaschrot
5,2 % Maiskleberfutter
5,2 % Weizenbollmehl
3,1 % Fischmehl
3,1 % Topiokamehl
3,1 % Luzernegrasgrünmehl
2,1 % Weizenkeime (zerkleinert)
1,7 % Fleichknochenmehl
1,6 % Molkenpulver
1,4 % kohlens. Futterkalk
1,0 % phosphors. Futterkalk
1,0 % Melasse.

Tabelle b

| Behandlung ppm | Gewichts- zuwachs | Futter- verbrauch | Futter- verwertung |
|---|---|---|---|
| 10 | 108 % | 97 % | 90 % |
| 25 | 108 % | 93 % | 86 % |

Le A 23 433

## Patentansprüche

1. Organisch chemische Verbindung gekennzeichnet durch

    a. IR-KBr-Absorptionsspektrum des natriumhaltigen
       Antibiotikums bei folgenden Wellenlängen
       (ausgedrückt in $cm^{-1}$) mit charakteristischen
       Absorptionsbanden

|      |      |
|------|------|
| 3456 | 1265 |
| 2960 | 1178 |
| 2910 | 1145 |
| 1750 | 1115 |
| 1700 | 1030 |
| 1635 |  950 |
| 1540 |  930 |
| 1395 |  825 |

    (vgl. Abb. 1, Abszisse: Wellenzahl in $cm^{-1}$;
    Ordinante: Absorption);

    b. $^1$H-Kernresonanzspektrum gemäß Abb. 2, angegeben in Teilen pro Million und Schwingungen
       pro Sekunde;

    c. ·Das 13C-Kernresonanzspektrum gemäß Abb. 3;

Le A 23 433

d. Elementaranalyse (nach 2 Tagen Trocknen im Hochvakuum bei 30°C): C 61,1 - 62,3 %; H 5,2 - 6,4 %; O 27,5 - 30,0 %;

e. Summenformel: $(C_{14-16}, H_{16-20}, O_{4-6})_n$;

f. Summenformel der natriumhaltigen Verbindung $(C_{29-31}, H_{33-37}, O_{9-11}, Na)$;

g. Löslichkeit:

Die Verbindung ist gut löslich in Essigsäureethylester, Chloroform, MeOH und niedrigen Alkoholen; schlecht löslich in Wasser;

h. Färbbarkeit:

Die Verbindung läßt sich nach Chromatographie auf Kieselgel-Dünnschichtplatten mit $FeCl_3/H_2SO_4$ anfärben.

2. Verfahren zur Herstellung der organisch chemischen Verbindung gemäß Anspruch 1, dadurch gekennzeichnet, daß man Mikroorganismen der Familie Streptomycetaceae insbeondere der Gattung Streptomyces und vorzugsweise den Streptomyces-Stamm BA 9 (entsprechend DSM Nr. 3025) in einem Nährmedium welches assimilierbare Kohlenstoff- und Stickstoffquellen sowie Mineralsalze enthält, unter aeroben Bedingungen kultiviert und die gewünschte Verbindung isoliert.

Le A 23 433

3. Organisch-chemische Verbindung gemäß Anspruch 1, erhältlich nach dem Verfahren gemäß Anspruch 2.

4. Antibiotikum gekennzeichnet durch den Gehalt an der Verbindung wie sie in den Ansprüchen 1-3 gekennzeichnet ist.

5. Prämixe und/oder Futterzusatzmittel gekennzeichnet durch den Gehalt an der Verbindung wie die in den Ansprüchen 1-3 gekennzeichnet ist.

6. Verwendung der Verbindung, wie sie in den Ansprüchen 1-3 gekennzeichnet ist, bzw. des Antibiotikums gegen gram-positive und gram-negative Keime als Therapeutikum.

7. Verwendung der Verbindung, wie sie in den Ansprüchen 1-3 gekennzeichnet ist bzw. der Prämixe und/oder Futterzusatzmittel gemäß Anspruch 5 zur Förderung und Beschleunigung des Wachstums bei Tieren, sowie zur Verbesserung der Futterverwertung bei Tieren.

8. Mikroorganismen der Familie Streptomycetaceae, insbesondere der Gattung Streptomyces, die bei einer Kultivierung in einem Kohlenstoff- und Stickstoffquellen sowie Mineralsaze enthaltendem Nährmedium die Verbindung, wie sie in den Ansprüchen 1 und 2 gekennzeichnet ist, produzieren.

Le A 23 433

9. Streptomyces-Stamm BA 9 (gemäß DSM Nr. 3025) sowie seine Mutanten und Varianten.

10. Verwendung von Mikroorganismen gemäß den Ansprüchen 8 und 9 zur Herstellung der Verbindung, wie sie in den Ansprüchen 1 und 2 gekennzeichnet ist.

Le A 23 433

FIG.1

FIG. 2

~CH₃OH

~CH₃OH

THS

0183214

8    7    6    5    4    3    2    1    0  PPM

FIG. 3

200   180   160   140   120   100   80   60   40   20   0 PPM